# FASCICULE DE BREVET EUROPEEN

(11) **EP 1 136 056 B1**
(45) Date de publication et mention de la délivrance du brevet: **10.06.2009**
(21) Numéro de dépôt: 01400399.0
(22) Date de dépôt: 15.02.2001
(51) Int. Cl.: A45D 34/04, A61K 8/02, A61K 8/86, A61Q 17/00, A61Q 19/00, B05B 11/00, B65D 35/36, B65D 47/42, B65D 81/28

(54) **Dispositif de conditionnement et d'application comportant une structure poreuse incorporant un agent biocide**
Vorrichtung zum Aufbewahren und Auftragen mit einem biozidhaltigen porösen Element
Device for storing and applying comprising a porous structure containing a biocidal agent

(30) Priorité: 16.02.2000 FR 0001902
(43) Date de publication de la demande: 26.09.2001
(73) Titulaire: L'ORÉAL, 75008 Paris (FR)
(72) Inventeur: Gueret, Jean-Louis, 75016 Paris (FR)
(74) Mandataire: Tanty, François

(56) Documents cités:
- EP-A- 0 302 575
- DE-A- 3 438 186
- FR-A- 2 588 835
- US-A- 5 013 459
- US-A- 5 105 993
- US-A- 5 154 325

## Description

La présente invention concerne les dispositifs de conditionnement et d'application d'un produit cosmétique, comportant un récipient pouvant être fermé de manière étanche, apte à contenir le produit et une structure poreuse sous forme d'une mousse ayant des cellules ouvertes pour l'application du produit.

Il existe un besoin pour éviter d'introduire des conservateurs dans le produit, notamment lorsque ce dernier est destiné au soin de zones sensibles, par exemple le visage.

L'invention vise à améliorer les dispositifs de conditionnement et d'application connus, utilisant une structure poreuse pour l'application, afin de réduire autant que possible la quantité de conservateur(s) dans le produit.

L'invention y parvient grâce au fait que le dispositif de conditionnement et d'application comporte au moins un agent biocide incorporé dans la structure poreuse.

Avantageusement, cet agent biocide se présente, lorsque la structure poreuse est sèche, à l'état solide ou concentré.

Grâce à l'invention, on empêche la structure poreuse de sécher, de par la fermeture étanche du récipient, et l'on fait en sorte qu'elle garde une humidité résiduelle permettant d'activer l'agent biocide.

Autrement dit, dans l'invention, l'agent biocide est libéré au sein de la structure poreuse pour l'empêcher de se dégrader.

De plus, la structure poreuse n'est sollicitée le plus souvent, dans la majorité des cas, qu'en surface essentiellement, durant l'application du produit ou l'essorage de l'applicateur, de sorte que l'agent biocide présent dans la structure poreuse tend à rester contenu dans celle-ci et ne se retrouve qu'à l'état de traces dans le produit déposé sur l'utilisateur, ce qui est avantageux lorsque le produit est destiné à des zones sensibles.

Par ailleurs, le fait que la structure poreuse soit empêchée de sécher permet d'éviter une alternance d'états secs et saturés d'humidité.

Or, cette alternance est souvent la cause principale du développement des moisissures et autres micro-organismes.

Ainsi, grâce au fait que dans l'invention la structure poreuse ne sèche pas, l'agent biocide peut être présent dans une quantité relativement faible.

Dans une réalisation préférée, le dispositif est agencé de telle sorte que la structure poreuse ne soit saturée de produit qu'en surface lors de son utilisation.

Cela permet d'éviter un lessivage de la structure poreuse par le produit à chaque utilisation, de sorte que l'on réduit le passage en solution de l'agent biocide.

Dans une réalisation préférée, la structure poreuse est hydrophile ou comporte un ou plusieurs agents biocides hydrophiles.

Le fait que la structure poreuse soit hydrophile favorise une humectation complète par le produit, lorsque ce dernier est aqueux.

En variante, la structure poreuse est lipophile et comporte un ou plusieurs agents biocides lipophiles.

La structure poreuse peut aussi comporter à la fois des agents biocides hydrophiles et des agents biocides lipophiles.

Comme agent(s) biocide(s), on peut utiliser un ou plusieurs agents bactéricides et/ou bactériostatiques et/ou antifongiques.

On peut également utiliser des sels métalliques.

La structure poreuse est telle que définit dans la revendication 1, et comporte une mousse réalisée à partir de polyéther, et comporte au moins 10 % de cellules ouvertes.

La structure poreuse peut présenter une structure composite avec plusieurs couches de nature différentes, par exemple plusieurs types de mousses.

Le produit cosmétique ou de soin utilisé comporte par exemple entre 0,5 et 95 % d'eau et peut être un gel, une émulsion, notamment une émulsion d'huile dans de l'eau ou d'eau dans de l'huile.

Le dispositif de conditionnement et d'application selon l'invention peut servir au maquillage, au traitement ou au soin de la peau ou des cheveux.

Un procédé pour assurer la conservation d'une structure poreuse utilisée dans le domaine cosmétique ou du soin, est caractérisé par le fait qu'il comporte les étapes consistant à incorporer, lors de la fabrication de la structure poreuse, au moins un agent biocide et à faire en sorte que la structure poreuse ne puisse sécher, entre deux utilisations, en l'enfermant dans un dispositif de conditionnement et d'application étanche ou sensiblement étanche.

De préférence, dans ce procédé, l'agent biocide se présente à l'état solide ou concentré au terme du processus de fabrication de la structure poreuse, lorsque cette dernière est sèche.

Ainsi, l'agent biocide se présente sous forme de cristaux par exemple, au sein de la structure poreuse. Ces cristaux assurent la protection de la structure poreuse contre les risques de dégradation en se solubilisant très progressivement au sein de la structure poreuse.

Un procédé de fabrication d'une structure poreuse, utilisée pour l'application d'un produit cosmétique ou de soin est caractérisé par le fait que l'on incorpore dans la structure poreuse durant son processus de fabrication au moins un agent biocide au moins partiellement hydrosoluble, de telle manière que cet agent se retrouve à l'état solide ou concentré lorsque la structure poreuse est sèche.

D'autres caractéristiques et avantages de la présente invention apparaîtront à la lecture de la description détaillée qui va suivre, d'exemples de mise en oeuvre et à l'examen du dessin annexé, sur lequel :
- la figure 1 est une vue schématique en coupe axiale d'un premier exemple de dispositif de conditionnement et d'application non-conforme à l'invention.
- la figure 2 est une vue schématique en coupe axiale d'un deuxième exemple de dispositif de conditionnement et d'application,
- la figure 3 est une vue schématique en coupe axiale d'un troisième exemple de dispositif de conditionnement et d'application non-conforme à l'invention,
- la figure 4 est une vue schématique en coupe axiale d'un quatrième exemple de dispositif de conditionnement et d'application,
- la figure 5 est une vue schématique en coupe axiale d'un cinquième exemple de dispositif de conditionnement et d'application selon l'invention non-conforme à l'invention, et
- la figure 6 est une vue en perspective d'un dispositif de conditionnement et d'application conforme à un sixième exemple de réalisation de l'invention.

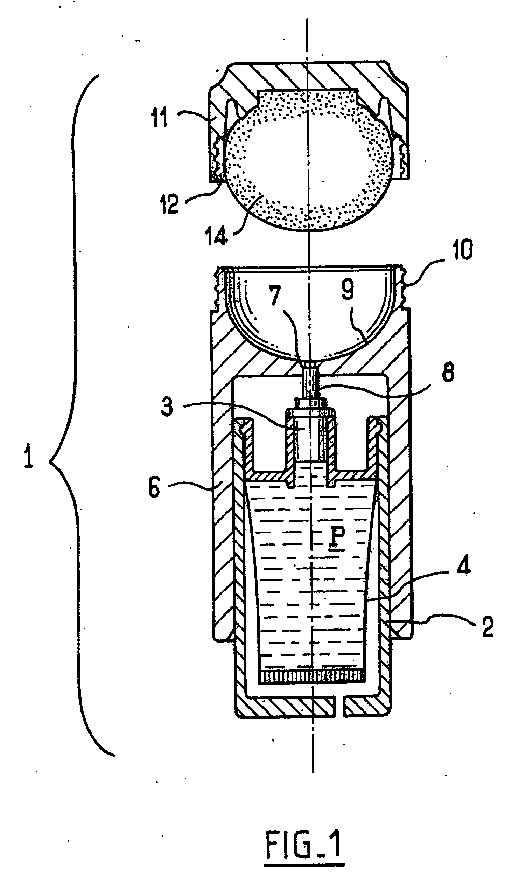

On a représenté sur la figure 1 un dispositif de conditionnement et d'application 1 comportant un corps 2 sur lequel est montée une pompe 3 sans reprise d'air. Cette pompe 3 est agencée pour pomper un produit aqueux P, cosmétique ou de soin, contenu dans une poche souple 4 logée dans le corps 2.

Un capot coulissant 6 est monté sur le corps 2. Ce capot 6 comporte en partie supérieure un col 10 fileté extérieurement et à l'intérieur de ce dernier un logement 9 sensiblement hémisphérique, ouvert vers le haut.

Le dispositif de conditionnement et d'application 1 comporte également un capot de fermeture 11, pourvu d'une jupe de montage 12 agencée pour se visser sur le col fileté 10.

Un applicateur 14, constitué par une mousse de caoutchouc naturel (NRB) à cellules ouvertes, est fixé à l'intérieur du capot de fermeture 11 et dépasse de celui-ci vers le bas afin de pouvoir être appliqué sur la peau.

Le capot de fermeture 11 sert d'organe de préhension.

Le capot 6 est traversé, dans le fond du logement 9, par un orifice comportant un épaulement 7 contre lequel vient en appui la tige 8 de la pompe 3.

Le col fileté 10 et la jupe de montage 12 sont agencés pour coopérer de manière étanche lorsque le capot de fermeture 11 est en place, de sorte que l'applicateur 14 est alors empêché de sécher.

L'applicateur 14 comporte dans sa masse un agent biocide.

Cet agent biocide est sous forme particulaire lorsque l'applicateur 14 est sec.

L'agent biocide utilisé est de plus dans l'exemple décrit au moins partiellement hydrosoluble.

D'une manière générale, comme agent biocide, on peut utiliser dans le cadre de la présente invention de l'acide salicylique, du sulfite de sodium, de l'acide borique, du méta-bi-sulfite de sodium, de l'acide benzoïque, du benzoate de sodium, du p-hydroxybenzoate de propyle, du p-hydroxybenzoate de méthyle, du p-hydroxybenzoate de méthyle sodé, du sulfate d'hydroxy-b-quinolénine, du bromure de myristyl tri-méthylammonium, du o-phényl phénate de sodium, du chlorure de stéaryl di-méthyl benzylammonium, de l'acide sorbique, un mélange de p-hydroxybenzoate de méthyle, édétate di-potassique, di-iséthionate d'hexamidine (58/30/12), du chlorhydrate de chlorhexidine, un mélange de p-hydroxybenzoate de propyle, méthyle, butyle, éthyle (7/57/22/14), du déhydroacétate de sodium hydraté, du sorbate de potassium, du trichloro-2,4,4' hydroxy-2' diphényléther, du chloracétamide, du di-iséthionate du di-(amidino-4 phénoxy)-1,6 hexane, un mélange de p-hydroxybenzoates de méthyle, éthyle, propyle (74/17/9), un chlorure de (chloro-3 allyl-1 tri-azo-3,5,7 azonium-1 adamante, un mélange de p-hydroxybenzoates de méthyle, propyle, bromo-2 nitropropane-2-di-ol-1,3, di-iséthionate d'hexamidine (76/2/3/17), mélange de pyrithione di-sulfure, sulfate de magnésium, de l'imidazolidinyl urée, du p-hydroxybenzoate de butyle, du p-hydroxybenzoate d'éthyle, du bromo-2 nitropropane-2 di-ol-1,3, du thiosalicylate d'éthyl mercure sodique, un mélange de chlorures d'alkyl di-méthyl benzyl ammonium, un mélange d'acide déhydroacétique, tri-chloro-2,4,4' hydroxy-2' di-phényléther, p-hydroxybenzoate de propyle (34/33/33), un mélange de p-hydroxybenzoates de méthyle, propyle, di-iséthionate d'hexamidine (80/3/17), de l'iodure d'(heptyl-2 méthyl-1 thiazolyn-2 ylidène-2)-2,2 méthylène heptyl-3 méthyl-2 thiazolynium, de l'hydroxy-1, méthyl-4, tri-méthyl pentyl-6 pyridone-2, sel de mono-ethanolamine, de l'acide déhydroacétique, de l'iso-propyl méta-crésol, du cyclo-hepta tri-en-2,4,6 one-1 hydroxy-2 (méthyl-1 éthyl-4), du N-(hydroxy-méthyl) N-(di-hydroxy-méthyl-1,3 di-oxo-2 imidazolidinyl-4) N-(hydroxy-méthyl urée), un mélange de chlorphénésine, di-iséthionate d'hexamédine, para-hydroxybenzoates de méthyle, butyle, propyle (75/15/7, 5/1/1.5), du para-chloro-méta-xylénol, un mélange de p-hydroxybenzoate de butyle, tri-chloro-2,4,4' hydroxy-2' di-phényl éther (50/50), un mélange de di-méthylol di-méthyl hydantoine, iodo-3-propinyl-2 carbamate (95/5), un mélange d'acide borique, di-iséthionate d'hexamidine, p-hydroxybenzoate de méthyle, bronopol (73,1/8,5/15/3,4), de l'iodure de (bromo-5 pyridyl-2) amino) vinyl-2)-p-éthyl-1 picolinium-2, un mélange d'imidazolidinyl urée, di-iséthionate d'hexamidine (85,7 15/14,285), un mélange de p-hydroxybenzoate de méthyle, di-iséthionate d'hexamidine (80/20), un mélange de di-azolidinyl urée, p-hydroxybenzoates de méthyle, propyle, di-iséthionate d'hexamidine (50/28/12/10), du chlorphénésine, un mélange de chlorphénésine, p-hydroxybenzoates de méthyle, propyle (50/41, 66/8, 34), un mélange de chlorphénisine, p-hydroxybenzoate de méthyle (60/40), du chlorure de béhényl tri-méthyl ammonium, du parahydroxybenzoate d'éthyle sodé.

Cette liste n'est pas limitative.

On peut également incorporer dans la mousse, outre un ou plusieurs agents biocides choisis dans la liste ci-dessus, des sels métalliques, par exemple d'argent ou de cuivre ou des oxydes ferriques.

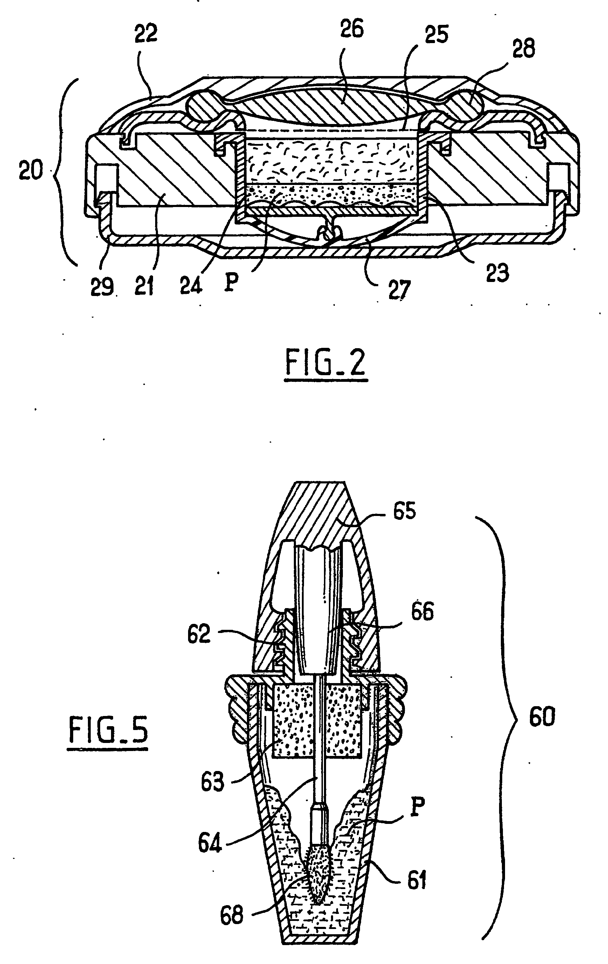

On a représenté sur la figure 2 un dispositif de conditionnement et d'application 20 comportant un boîtier ayant un corps 21, un couvercle articulé 22 et un fond coulissant 29.

Le corps 21 comporte un logement recevant une coupelle 23 contenant une réserve de produit P.

La coupelle 23 est munie en partie supérieure d'un tamis 25 et comporte en partie inférieure une paroi déformable 27.

Un piston 24 solidaire de la paroi déformable 27 peut coulisser dans la coupelle 23.

En appuyant sur le fond coulissant 29, l'utilisateur repousse le piston 24 vers le haut et chasse du produit à travers le tamis 25.

Un applicateur 26 constitué par une éponge se loge à l'intérieur du couvercle 22 au-dessus du tamis 25.

Le dispositif de conditionnement et d'application 20 est agencé pour assurer la conservation étanche de l'applicateur 26.

A cet effet, l'applicateur 26 est solidaire à sa périphérie d'un bourrelet annulaire 28 qui sert de joint d'étanchéité en étant pincé lorsque le boîtier est fermé entre le couvercle 22 et un cache monté sur le corps 21.

L'applicateur 26 est ainsi empêché de sécher lorsque le couvercle 22 est fermé.

L'applicateur 26 incorpore un agent biocide, par exemple un agent biocide choisi dans la liste ci-dessus.

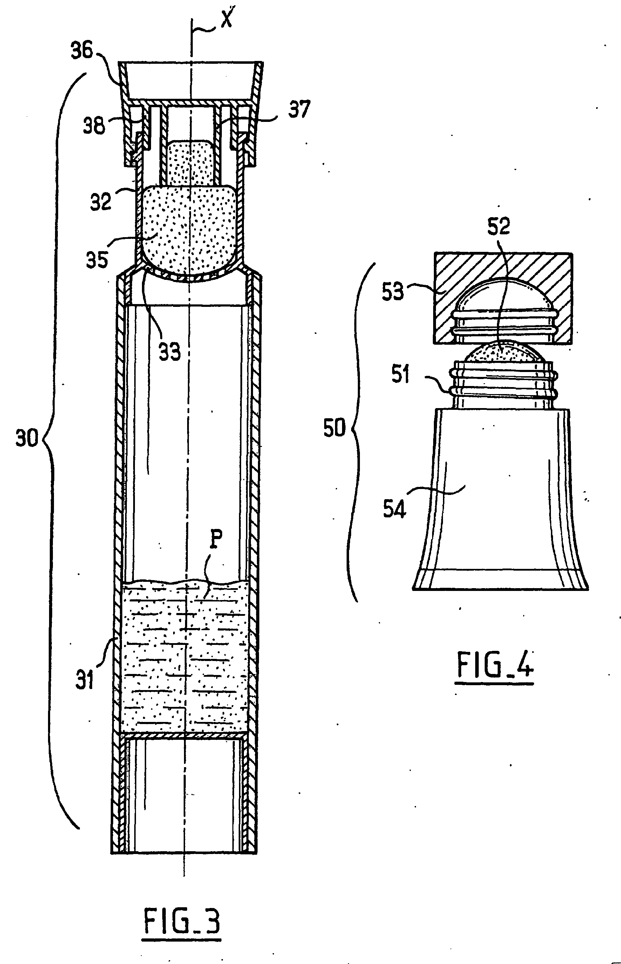

On a représenté sur la figure 3 un dispositif de distribution et de conditionnement 30 qui comporte un récipient 31 pourvu d'un col fileté extérieurement 32.

Le col 32 comporte intérieurement, dans son fond, une paroi perforée 33.

Le dispositif de distribution et de conditionnement 30 comporte un applicateur 35 porté par un organe de préhension 36 qui constitue également le capuchon de fermeture du col 32.

L'applicateur 35 est partiellement engagé et fixé dans une jupe de montage 37 du capuchon 36.

Le capuchon 36 comporte une jupe d'étanchéité 38 conformée pour s'ajuster de manière étanche dans le col 32 lorsque le capuchon 36 est vissé sur le col 32.

L'applicateur 35 est constitué par une mousse de polyuréthane à cellules ouvertes et incorpore un agent biocide choisi dans la liste ci-dessus, présent sous forme particulaire ou cristallisée lorsque la mousse est sèche.

On a représenté sur la figure 4 un dispositif de distribution et de conditionnement 50 comprenant un tube souple 54 pourvu d'un col fileté extérieurement 51, ce col étant muni intérieurement d'un applicateur 52 constitué par un bloc de mousse de polyéther à cellules ouvertes.

L'applicateur 52 incorpore un agent biocide choisi dans la liste ci-dessus, présent sous forme particulaire ou cristallisée lorsque la mousse est sèche.

En l'absence d'utilisation, le tube 54 est fermé de manière étanche par un capuchon de fermeture 53.

On a représenté sur la figure 5 un dispositif de conditionnement et d'application 60 comportant un récipient 61 pourvu d'un col 62 fileté extérieurement.

Un organe d'essorage 63 constitué par un bloc de mousse de polyuréthane à cellules ouvertes, fendu axialement, est fixé à l'intérieur du récipient 61 sous le col 62.

Le récipient 61 est fermé par un capuchon de fermeture 65 vissé sur le col 62.

Ce capuchon de fermeture 65 comporte une partie centrale 66 apte à s'ajuster de manière étanche dans le col 62.

La partie centrale 66 se prolonge vers le bas par une tige 64 pourvue en extrémité d'un applicateur floqué 68.

L'organe d'essorage 63 sert à essorer la tige 64 et l'applicateur 68 lorsque ce dernier est extrait du récipient 61.

Il incorpore un agent biocide choisi dans la liste ci-dessus, présent sous forme particulaire ou cristallisée lorsque la mousse est sèche.

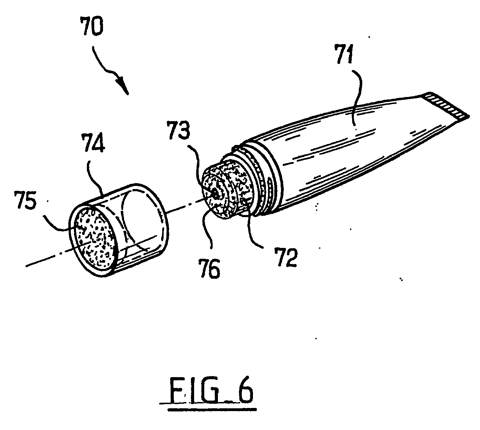

On a représenté sur la figure 6 un dispositif de conditionnement et d'application 70 conforme à un sixième exemple de réalisation.

Ce dispositif comporte un tube 71 à parois souples, contenant par exemple une base de maquillage telle qu'un lait cosmétique.

Le tube 71 est pourvu d'un organe d'application 72 élastiquement déformable, qui incorpore un agent biocide choisi dans la liste ci-dessus.

L'organe d'application 72 est traversé par un orifice 73 qui permet au produit contenu dans le tube de gagner facilement la surface 76 servant à l'application du produit sur l'utilisateur.

Le dispositif 70 comporte un capuchon de fermeture 74, réalisé dans une matière plastique transparente, qui présente la particularité de contenir une réserve 75 d'un produit différent de celui contenu dans le tube.

Ce produit est constitué, dans l'exemple décrit, par un pain de produit coloré, apte à se déliter au contact du produit contenu dans le tube.

Lorsque le capuchon est monté sur le tube, la surface d'application 76 vient en appui sur la réserve du produit.

Le capuchon 74 est capable de fermer de manière étanche le tube lorsqu'il est en place.

Dans tous les exemples de réalisation qui viennent d'être décrits, l'applicateur ou l'organe d'essorage sont conservés dans une enceinte étanche ou sensiblement étanche en l'absence d'utilisation, de sorte que l'applicateur ou l'organe d'essorage ne peuvent sécher totalement et conservent une humidité résiduelle qui permet d'activer le ou les agents biocides qu'ils contiennent.

L'applicateur ou l'organe d'essorage sont ainsi préservés de toute dégradation par des micro-organismes.

Du fait de cette conservation du matériau alvéolaire constituant l'applicateur ou l'organe d'essorage, il est possible d'abaisser la teneur en conservateur(s) dans le produit, puisque ce dernier n'a pas à assurer la fonction de conservation de ce matériau alvéolaire, mais doit simplement participer à l'activation du ou des agents biocides contenus à l'intérieur.

On incorpore un agent biocide dans le matériau alvéolaire lors de sa fabrication, c'est-à-dire lors de l'étape de moussage de la matière plastique.

On peut utiliser, sans sortir du cadre de la présente invention, le matériau alvéolaire contenant l'agent biocide comme une réserve de produit, en l'imbibant de produit.

Dans tous les exemples de réalisation décrits, le matériau alvéolaire peut être floqué ou non et contenir plus ou moins de cellules ouvertes.

Le matériau alvéolaire peut recevoir un traitement hydrophile postérieurement à sa fabrication ou lors de sa fabrication, par incorporation d'hydroabsorbants ou de fibres, par exemple du polyacrylate, de la silice colloïdale, des alginates, de la glycérine, des fibres de coton, de l'amidon, cette liste étant bien entendu non-limitative.

## Revendications

1. Dispositif de conditionnement et d'application pour le conditionnement et l'application d'un produit cosmétique, comportant un récipient pour contenir le produit et pouvant être fermé de manière étanche et une structure poreuse comportant une mousse en matière plastique alvéolaire pour l'application du produit **caractérisé par le fait que** la mousse est réalisée à partir de polyéther et comporte au moins 10 % de cellules ouvertes et comporte au moins un agent biocide incorporé dans la matière plastique durant la fabrication de la structure poreuse lors de l'étape de moussage de la matière plastique.

2. Dispositif selon la revendication précédente, **caractérisé par le fait que** l'agent biocide se présente, lorsque la structure poreuse est sèche, à l'état solide ou concentré.

3. Dispositif selon la revendication 1 ou 2, **caractérisé par le fait qu'**il est agencé de telle sorte que la structure poreuse ne soit saturée de produit qu'en surface, lors de son utilisation.

4. Dispositif selon l'une des revendications précédentes, **caractérisé par le fait que** la structure poreuse est hydrophile, respectivement lipophile et comporte un ou plusieurs agents biocides hydrophiles, respectivement lipophiles.

5. Dispositif selon l'une quelconque des revendications précédentes, **caractérisé par le fait que** l'on utilise comme agent biocide un agent bactéricide et/ou bactériostatique et/ou antifongique.

6. Dispositif selon l'une quelconque des revendications précédentes, **caractérisé par le fait que** l'on utilise un ou plusieurs agents biocides hydrosolubles.

7. Dispositif selon l'une quelconque des revendications 1 à 5, **caractérisé par le fait que** l'on utilise un ou plusieurs agents biocides liposolubles.

8. Dispositif selon l'une quelconque des revendications précédentes, **caractérisé par le fait que** la structure poreuse comporte des sels métalliques.

9. Dispositif selon l'une quelconque des revendications précédentes, **caractérisé par le fait que** le produit comporte entre 0,5 et 95 % d'eau.

10. Dispositif selon l'une quelconque des revendications précédentes, **caractérisé par le fait que** le produit est un produit de maquillage, de traitement ou de soin de la peau ou des cheveux.

## Claims

1. A packaging and applicator device for packaging and applying a cosmetic product, the device comprising a receptacle containing the substance being configured for being closed in sealed manner, and a porous structure comprising a foam for applying the substance, the device being **characterized by** the fact that the foam is made from polyether and comprises at least 10% open cells and comprises at least one biocidal agent incorporated in the porous structure during the process of manufacture of the porous structure.

2. A device according to the preceding claim, **characterized by** the fact that, when the porous structure is dry, the biocidal agent is present in the solid or concentrated state.

3. A device according to claim 1 or 2, **characterized by** the fact that while it is in use, the porous structure is arranged in such a manner as to be saturated with the substance on its surface only.

4. A device according to any preceding claim, **characterized by** the fact that the porous structure is hydrophilic, or lipophilic, and comprises one or more biocidal agents that are correspondingly hydrophilic or lipophilic.

5. A device according to any preceding claim, **characterized by** the fact that the biocidal agent used is a bactericidal agent and/or a bacteriostatic agent and/or an antifungal agent.

6. A device according to any preceding claim, **characterized by** the fact that one or more hydrosoluble biocidal agents are used.

7. A device according to any one of claims 1 to 5, **characterized by** the fact that one or more liposoluble biocidal agents are used.

8. A device according to any preceding claim, **characterized by** the fact that the porous structure includes metallic salts.

9. A device according to any preceding claim, **characterized by** the fact that the substance contains 0.5% to 95% water.

10. A device according to any preceding claim, **characterized by** the fact that the substance is a makeup, a treatment substance or a care product for the skin or the hair.

## Patentansprüche

1. Verpackungs- und Anwendungsvorrichtung für die Verpackung und Anwendung eines kosmetischen Produkts, die einen Behälter, der das Produkt enthält und dicht verschlossen werden kann, und eine poröse Struktur mit einem wabenförmigen Schaumstoff für die Anwendung des Produkts umfasst, **dadurch gekennzeichnet, dass** der Schaumstoff aus Polyether hergestellt ist, wenigstens 10 % offene Zellen enthält und wenigstens ein Biozidmittel enthält, das während der Herstellung der porösen Struktur im Schritt des Aufschäumens des Kunststoffs in den Kunststoff eingearbeitet wird.

2. Vorrichtung nach dem vorhergehenden Anspruch, **dadurch gekennzeichnet, dass** das Biozidmittel dann, wenn die poröse Struktur trocken ist, im festen oder konzentrierten Zustand vorliegt.

3. Vorrichtung nach Anspruch 1 oder 2, **dadurch gekennzeichnet, dass** sie so beschaffen ist, dass die poröse Struktur bei ihrer Verwendung nur an der Oberfläche mit dem Produkt gesättigt ist.

4. Vorrichtung nach einem der vorhergehenden Ansprüche, **dadurch gekennzeichnet, dass** die poröse Struktur hydrophil bzw. lipophil ist und ein oder mehrere hydrophile bzw. lipophile Biozidmittel enthält.

5. Vorrichtung nach einem der vorhergehenden Ansprüche, **dadurch gekennzeichnet, dass** als Biozidmittel ein bakterizides und/oder bakterienhemmendes und/oder pilzhemmendes Mittel verwendet wird.

6. Vorrichtung nach einem der vorhergehenden Ansprüche, **dadurch gekennzeichnet, dass** ein oder mehrere wasserlösliche Biozidmittel verwendet werden.

7. Vorrichtung nach einem der Ansprüche 1 bis 5, **dadurch gekennzeichnet, dass** ein oder mehrere fettlösliche Biozidmittel verwendet werden.

8. Vorrichtung nach einem der vorhergehenden Ansprüche, **dadurch gekennzeichnet, dass** die poröse Struktur metallische Salze enthält.

9. Vorrichtung nach einem der vorhergehenden Ansprüche, **dadurch gekennzeichnet, dass** das Produkt Wasser im Bereich von 0,5 bis 95 % enthält.

10. Vorrichtung nach einem der vorhergehenden Ansprüche, **dadurch gekennzeichnet, dass** das Produkt ein Schminkprodukt oder ein Produkt zur Behandlung oder Pflege der Haut oder der Haare ist.
